(19)

Europäisches Patentamt

European Patent Office

Office européen des brevets

(11) **EP 4 407 298 B1**

(12) **EUROPEAN PATENT SPECIFICATION**

(45) Date of publication and mention
of the grant of the patent:
**24.12.2025  Bulletin 2025/52**

(21) Application number: **23153737.4**

(22) Date of filing: **27.01.2023**

(51) International Patent Classification (IPC):
**G01N 21/35** *(2014.01)*      **G01N 21/84** *(2006.01)*
**G01N 21/3563** *(2014.01)*   **G01N 21/359** *(2014.01)*
**G01N 33/02** *(2006.01)*      **G01N 21/39** *(2006.01)*
**G01N 21/65** *(2006.01)*      **G01N 33/15** *(2006.01)*

(52) Cooperative Patent Classification (CPC):
**G01N 21/65; G01N 21/35;** G01N 21/3563;
G01N 21/359; G01N 33/02; G01N 33/15;
G01N 2021/8411

(54) **SYSTEMS AND METHODS FOR TESTING CONFORMITY OF SAMPLES USING SPECTROSCOPIC MEASUREMENTS**

SYSTEME UND VERFAHREN ZUM TESTEN DER ÜBEREINSTIMMUNG VON PROBEN MIT SPEKTROSKOPISCHEN MESSUNGEN

SYSTÈMES ET PROCÉDÉS POUR TESTER LA CONFORMITÉ D'ÉCHANTILLONS À L'AIDE DE MESURES SPECTROSCOPIQUES

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(43) Date of publication of application:
**31.07.2024  Bulletin 2024/31**

(73) Proprietor: **Bruker Optics GmbH & Co. KG**
**76275 Ettlingen (DE)**

(72) Inventor: **Niemoeller, Andreas**
**Ettlingen (DE)**

(74) Representative: **Bittner, Peter et al**
**Peter Bittner und Partner**
**Herrenwiesenweg 2**
**69207 Sandhausen (DE)**

(56) References cited:
- **PESTIEAU AUDE ET AL:** "Towards a real time release approach for manufacturing tablets using NIR spectroscopy", JOURNAL OF PHARMACEUTICAL AND BIOMEDICAL ANALYSIS, vol. 98, 2014, AMSTERDAM, NL, pages 60 - 67, XP093062133, ISSN: 0731-7085, DOI: 10.1016/j.jpba.2014.05.002

- **CAI C B ET AL:** "Treating NIR data with orthogonal discrete wavelet transform: Predicting concentrations of a multi-component system through a small-scale calibration set", TALANTA, ELSEVIER, AMSTERDAM, NL, vol. 77, no. 2, 15 December 2008 (2008-12-15), pages 822 - 826, XP025586409, ISSN: 0039-9140, [retrieved on 20080731], DOI: 10.1016/J.TALANTA.2008.07.037
- **DEPCZYNSKI U ET AL:** "Quantitative analysis of near infrared spectra by wavelet coefficient regression using a genetic algorithm", CHEMOMETRICS AND INTELLIGENT LABORATORY SYSTEMS, ELSEVIER SCIENCE PUBLISHERS B.V. AMSTERDAM, NL, vol. 47, no. 2, 17 May 1999 (1999-05-17), pages 179 - 187, XP004164072, ISSN: 0169-7439, DOI: 10.1016/S0169-7439(98)00208-1

EP 4 407 298 B1

## Description

## Technical Field

The present invention generally relates to spectroscopic measurements of samples, and more particularly relates to the testing of sample conformity utilizing spectra obtained by vibrational spectroscopy.

## Background

Vibrational spectroscopy is a method for direct measurement of covalent bonds in molecules consisting of atoms. Ionic bonds or atomic bonds (metals) are not relevant in this context. Vibrational spectroscopy methods include infrared spectroscopy - e.g., near-infrared (NIR) or mid-infrared (MIR) - and vibrational Raman spectroscopy.

An advantage of vibrational spectroscopy is that samples can be analyzed in a nonintrusive manner. Examples of common NIR applications are in the field of chemical analysis and address the quantitative analyses of chemical or physical properties of samples. For example, sample properties which can be analyzed with NIR/MIR spectroscopy include but are not limited to a concentration (in said sample) of: a particular compound in the measured sample, the iodine number, the pH value, the content of water, protein, fat, fiber, ash, fatty acids, and free fatty acids; or another property (measurable by NIR/MIR spectroscopy) not related to a concentration comprising: density, viscosity, octane and cetane number. Besides such quantitative analysis applications, there are applications where neither a sample product needs to be identified, nor its composition or other sample properties need to be quantified. Rather, such non-quantitative analysis applications are directed to qualitative and comparative analyses of a sample. Examples of such non-quantitative analyses include:

- Checking the conformity of a sample with reference samples: conformity is fulfilled when the composition of the sample and/or the characterizing properties of the sample correspond to the composition and/or characterizing properties of the reference samples within a predefined tolerance range.

- Detecting adulteration of a sample: adulteration may occur for example in the field of food products or medicaments. In particular in an untargeted (or non-targeted) approach, a search for deviations from reference samples is performed even without an assumption that a certain impurity is known or even present.

- Monitoring a chemical process: the goal is to detect deviations of the actual process (e.g., a chemical reaction or a fermentation) from a target process point and, in case of such deviations, trigger intervening process control commands, or to terminate the process when the end point is reached and no deviations from the target are found.

Prior art approaches addressing the above non-quantitative analyses are known, such as the conformity test described by Plugge and van der Vlies in "Near-infrared spectroscopy as an alternative to assess compliance of ampicillin trihydrate with compendial specifications", Journal of Pharmaceutical & Biomedical Analysis, 11435-442 (1993), or approaches based on Principle Component Analysis described for example by López et al. in "Multivariate screening in food adulteration: Untargeted versus targeted modelling", Food Chemistry, Volume 147, 2014, 177-181, https://doi.org/10.1016/j.foodchem.2013.09.139. These prior art approaches use algorithms or models which can confirm conformity of a sample by proving that there are no deviations detected from reference samples. However, the approaches are static in that a new spectrum of an unknown sample is always evaluated in the same way based on comparisons with a static set of reference spectra in a respective library. For dealing with samples of new sample types, the number of reference spectra increases over time to keep the method up to date. For example, new product variants need to be evaluated and a library needs to be expanded to cover such new samples due to, for example, different geographic origins, seasonal differences, or batch and supplier variations. But also changes in recipes or varying process flows or process conditions lead to a substantial increase in the number of necessary reference samples and their respective reference spectra. With such an increased number of reference spectra, the prior art approaches become less specific and less sensitive as the spectral variance in the library of reference spectra is increasing, but detecting new and unwanted spectral variance is an advantage of non-targeted approaches. In "Towards a real time release approach for manufacturing tablets using NIR spectroscopy" Journal of Pharmaceutical and Biomedical Analysis, Volume 98, 2014, Pages 60-67, ISSN 0731-7085, Aude Pestieau et al. disclose a classic conformity test for a powder mix and use a kNN-method for sample classification. In "Treating NIR data with orthogonal discrete wavelet transform: Predicting concentrations of a multi-component system through a small-scale calibration set", Talanta, Volume 77, Issue 2, 2008, Pages 822-826, ISSN 0039-9140, Cai et al. disclose that, through randomly arranging samples of a calibration set, treating their NIR spectra with orthogonal discrete wavelet transform, and selecting suitable variables in terms of correlation coefficient test, it is possible to extract features of each component in a multi-component system respectively and partial least squares models based on these features are capable of predicting the concentration of every component.

**Summary**

A dynamic evaluation which takes into account the particular situation of the sample (the actual sample properties) is not possible when using the prior art approaches. There is therefore a need to provide system and method that overcome the above-described drawbacks of the prior art allowing a continuous growth of a reference spectrum library to accommodate reference spectra for all kinds of new sample types and, at the same time, using a dynamic approach for the selection of appropriate reference spectra for highly specific and sensitive non-quantitative analyses. Such dynamic approach limits the spectral variance due to the localization of reference spectra to be used for a particular sample.

With the herein disclosed approach, untargeted (non-targeted) evaluations are possible for the non-quantitative analysis types described in the background section without the need to build a specific method for each new sample type. In accordance with "Appendix XVIII : USP 3 S ( FCC 11) Guidance on Developing and Validating Non-Targeted Methods for Adulteration Detection", 2019: "A non- targeted method consists of an analytical measurement that is sensitive to multiple potential adulterants coupled with a statistical model that recognizes deviations from the signal associated with the nominal material: it is not calibrated for any specific adulterants. Such methods have significant practical benefits but due diligence is required in their development, validation, and implementation to ensure sensitivity and specificity."

A library of reference spectra can be continuously extended with new reference spectra which allow to also represent a wide variety of products and process states with respective reference spectra. The herein disclosed approach uses a k-nearest neighbor (kNN) search to select a certain number of reference spectrum neighbors in the vicinity of a spectrum associated with a to-be-analyzed sample. As defined by David B. Hibbert in "Vocabulary of concepts and terms in chemometrics (IUPAC Recommendations 2016), Pure Appl. Chem. 2016; 88(4): 407-443 in section 6.20 k-nearest neighbor is a non-parametric method to find closest training examples in the variable space of reference spectra to analyze the sample. kNN approaches can be used further as a supervised classification method where an object is classified by a majority vote regarding the class of the most common nearest neighbors. In the herein disclosed approach, the search of kNN reference spectra can be limited to a class which is given with the sample to be analyzed.

This selection of reference spectra is then used as the basis for the evaluation of the sample with regard to reference samples represented by the selected reference spectra in accordance with the below described computer-implemented method. It is advantageous that new reference spectra and data sets can be easily added to the spectrum library, since a pure spectrum library is used without any underlying model.

For example, in the case of adulteration detection with untargeted prior art approaches, a product such as milk, which is subject to regional and seasonal fluctuations, cannot be covered over time with a simple method. Either many reference spectra of samples covering such natural variations are added which reduces the sensitivity of the adulteration detection, or more specific methods are used which easily tend to be too sensitive and provide false negative results. The kNN search approach in a reference spectrum library makes it possible to allow a large diversity of reference samples with precise testing.

In the example of process control, typically there are many effects in process sequences (or process flows), so that processes which are supposed to provide the same product typically deviate from the ideal target process path in particular with regard to the time axis. Further, typically the sample composition during the process changes permanently. In prior art approaches, respective modelling is therefore usually only carried out for the last part and the termination of a process. Such modeling is product-dependent and requires a method per product. Further, the prior art approaches do not allow monitoring of the entire process and check for unexpected deviations from the ideal process also in the early or middle phases of the process. The herein disclosed approach allows the consideration of sample spectra during the entire process (in whole or in part) for one or more products. Typically, the sample composition changes during the processing with each new sample composition corresponding to a new sample. That is, it is possible to check in the initial phase or anytime in the course of a process whether the process is running as specified by the respective reference spectra. The spectrum library comprises reference spectra for a plurality of different sample properties which occur during the processing of the sample. The kNN search can then identify, for each sub-process of the processing, an appropriate subset of respective reference spectra.

Embodiments of the herein disclosed dynamic approach in accordance with the invention, defined in the independent claims, include a computer-implemented method for determining conformity of a fingerprint of a sample with at least one corresponding expected fingerprint of said sample, a computer program product having computer readable instructions which, when executed by one or more processors of a computing device, cause the computing device to execute the herein disclosed computer-implemented method, and a computer system (data processing system) which can load the computer program product to implement modules which allow the computer system to execute the steps of the computer-implemented method. In short, a conformity test is disclosed for checking a sample by its spectral fingerprint. A respective measured spectrum in total is representing the sample with all its properties (mainly the total composition of the sample). The term measured spectrum as used herein may also refer to one or more sub-ranges of the entire spectrum where the respective one or more sub-ranges are only used for the setup of the conformity test and the evaluations by the conformity test. The conformity test provides a statistical approach to check if the measured spectrum matches with the spectral variance of the reference spectra (reference fingerprints) in a spectrum library associated with reference samples. In other words, the fingerprint is characteristic of the sample with regard to the sample's chemical or physical properties which cause a spectral contribution to the measured spectrum. Such example characteristics include, but are not limited to, the quality of the sample and the composition of the sample, dependent on the used technology (e.g., infrared, near-infrared or Raman). Advantageously, the spectral range(s) of the measured spectrum (either the range of the entire sample spectrum or the one or more sub-ranges corresponding to one or more sub-portions of the sample spectrum to be used for the conformity test) are used to select the corresponding range or sub-range(s) of the respective reference spectra in the spectrum library for the conformity test. This allows to use reference spectra covering different spectral ranges which can be useful in cases where reference spectra were obtained by different spectrometer devices which may cover different spectral ranges. Further, the setup of the conformity test can ensure that only reference spectra which provide data points in the respective spectral ranges (or sub-ranges) are used by the conformity test.

The computer-implemented method for determining conformity of a fingerprint of a sample with at least one corresponding expected fingerprint of said sample starts with obtaining a measured spectrum of the sample (the to-be-analyzed sample). The measured spectrum is the result of a measurement reflecting the chemical and physical properties (e.g., composition, density, viscosity, particle size, octane, cetane number, etc.) of the sample obtained by vibrational spectroscopy. The measured spectrum corresponds to the fingerprint of said sample. For example, the measured spectrum may be obtained by using infrared spectroscopy or by using Raman spectroscopy. Sample properties include, but are not limited to chemical composition, density, viscosity, particle size, octane and cetane number.

Then, a spectrum library is accessed which comprises a plurality of calibrated reference spectra. A calibrated reference spectrum is a spectrum which has been obtained from a reference sample where the conformity was proven by reference or testing methods according to the purpose of the conformity testing (batch conformity, quality testing, untargeted adulteration screening, mixing uniformity, process monitoring etc.). Thereby, the spectrum library may include reference spectra obtained from reference samples of different sample types. For the computer-implemented method it is not necessary that the reference samples are restricted to the sample type corresponding to the sample type of the to-be-analyzed sample. Rather, the library can include reference spectra for a large range of sample types while high specificity and high sensitivity of the method are preserved.

In the next step, a subset of the reference spectra is identified in the spectrum library. Thereby, the subset is identified by determining representations of k nearest neighbor (kNN) reference spectra in the vicinity of a corresponding representation of the measured spectrum in accordance with predefined metrics. For the kNN search, the system may use directly the spectral data of the measured spectrum and compare it to spectral data of the reference spectra in the reference library. Spectral data may be preprocessed by, e.g., baseline corrections, 1st or 2nd derivatives, normalization. For example, the search can be limited to reference spectra of a given class defined for the measured spectrum.

The k nearest neighbors may be identified based on the mathematical distance between the data points of the reference spectra and the respective data points of the measured spectrum. A data point of a spectrum corresponds to a wavenumber or wavelength with a measured spectrum value, e.g., an absorbance unit value. The mathematical distance is a measure for the distances between objects in multidimensional space. In general, the distance between any two points in n-dimensional space may be calculated by the Minkowski Metric. There are three special cases of the Minkowski distance: city block (Manhattan) distance, the Hamming distance, and the Euclidean distance. In practical experiments, the number k of nearest neighbors was selected from a range of 25 to 1000. Advantageously, the number k is selected from the range of 50 to 300.

In an optional embodiment, the representations of the measured spectrum and the reference spectra in the library may be obtained by applying a wavelet transformation to the respective spectra. Identifying of the subset is then performed in accordance with predefined metrics (e.g., using the Minkowski distance) in the wavelet space. In this embodiment, the wavelet transformation is used as a filter. That is, not the spectra but rather the wavelet-transformed spectra are evaluated, which has advantages such as enabling various filter and signal selection procedures that are not possible with prior art approaches. For example, the wavelet transformation may be a discrete wavelet transformation (DWT), but other wavelet transformation types (e.g., continuous or multiscale wavelet transform (CWT), wavelet package transformation) may be used as well. As a result, a sample set of wavelet coefficients in a plurality of wavelet bands can be computed. In general, a wavelet transformation allows to analyze a signal on contained frequencies but, in contrast to a Fourier Transformation, it is known where the frequency is located in the signal (cf. U. Depczynski, K. Jetter, K. Molt and A. Niemoeller, Quantitative analysis of near infrared spectra by wavelet coefficient regression using a genetic algorithm, Chemometrics and Intelligent Laboratory Systems 47, 179-187, 1999). The wavelet itself has a compact support, meaning that it is not periodic. A wavelet transformation of a spectrum results in a lowpass representation band and the so-called detailed bands. The wavelets coefficients of the lowpass representation band (j=0) are the so-called approximation or scaling coefficients. Such approximation coefficients are not providing valuable information for the later steps and are not used any further for the herein described approach. The valuable information about details of the signal is found in the wavelet coefficients in the detailed bands starting with the band j=1. In the following description, the band j=1 is always referred to as the lowest band.

There are several families with many possible wavelet types and shapes. For example, for the wavelet transformation, the Haar-Wavelet as the simplest one and the common other Daubechies types can be used to decompose a spectrum and to use the coefficients directly, as well as any other wavelet type including more complex ones. However, in the context of the herein disclosed approach the wavelet type is not too relevant but still may have influence on results and complexity of the described process. The wavelet transformation is fast and works with each individual spectrum alone. That is, any spectrum can be transformed individually into a corresponding wavelet-transformed spectrum to be used in a library. That is, in the wavelet space each calibration set can be added to the library or deleted from the library without affecting the other calibration sets of the library. For the wavelet transformation a pre-processing of the spectra is not required at all but can be done.

In particular, in the case of adulteration detection, the spectrum of the to-be-analyzed sample may show specific deviations and the kNN search performed directly on the spectral data may not lead to actually matching spectra. When performing the kNN search in the wavelet space, the kNN search may consider more or less details of the spectra depending on the selection of certain wavelet bands. That is, determining representations of k nearest neighbor reference spectra may only use a subset of available wavelet bands. The subset of available wavelet bands may comprise only wavelet bands with low and medium frequencies.

Optionally, the kNN search can be further accelerated by providing, in addition to the measured spectrum, information about the sample type (e.g., the product type or class associated with the to-be-analyzed sample - e.g., IUPAC reference -, or certain raw material included in the to-be-analyzed sample). In this embodiment, the reference spectra in the reference library are annotated with corresponding tags so that the plurality of reference spectra can be pre-filtered in accordance with the additionally provided information. The kNN search is then performed on the pre-filtered subset of reference spectra which can significantly speed up the kNN search.

Once the k nearest neighbors are identified via the wavelet coefficients which represent the respective spectra, the conformity test is then performed on the corresponding spectral data of the measured and reference spectra in full detail. It would be equivalent to perform the conformance test in the wavelet space when using all wavelet coefficients of the respective wavelet transformation results. However, the interpretation of the conformity test in the wavelet space is more difficult to represent and interpret.

The system now computes an averaged reference spectrum based on the identified subset, and further computes the standard deviation $\sigma_i$ (around the average absorbance value) in each data point i of the k nearest neighbor reference spectra. Then, a difference spectrum is determined by computing the absolute difference between the measured spectrum and the averaged reference spectrum divided by the corresponding standard deviation $\sigma_i$ in each data point i. That is, the difference spectrum describes the absolute difference between the measured spectrum and the averaged reference spectrum in each data point as a factor, which - when multiplied with the respective standard deviation - results in the absolute difference. This allows to determine deviating data points where the value of the difference spectrum exceeds a predefined deviation threshold. In other words, for each data point i an individual limit - the predefined deviation threshold - is defined by using the standard deviation $\sigma_i$ plus/minus around the average absorbance value. Thereby, the predefined deviation threshold is expressed as a multiple of the respective standard deviation, wherein the multiple can also be a fractional multiple (e.g., 3.4). Advantageously, the predefined deviation threshold is in the range from 3 to 8 based on the respective standard deviations.

Finally, the system determines conformity of the to-be-analyzed sample with the identified kNN reference samples in accordance with a predefined conformity rule based on the data points exceeding the predefined deviation threshold. In the following, three different conformity rules are shortly discussed which implement three different levels of strictness for the conformity assessment:

- The first conformity rule determines conformity if no deviating data point at all is determined. That is, according to the first rule, conformity is required over all data points (i.e., the entire frequency range) of the measured spectrum.
- The second conformity rule determines conformity if the average value of the values of the difference spectrum at the deviating data points is below a further threshold predefined for the second rule. That is, according to the second rule, deviating data points are still allowed for conformity as long as the average of the deviating values (the sum of the deviating values divided by the number of deviation data points) is below the further threshold for the second rule.
- The third conformity rule determines conformity if the sum of the values of the difference spectrum at the deviating data points divided by the total number of all data points is below a further threshold predefined for the third rule. That is, according to the third rule, the sum of the deviation values is divided by the number of all data points which is typically larger than the number of deviating data points. Therefore, in case the further threshold used for the third rule is equal to the further threshold used for the second rule, the tolerance for deviations which would still be seen as sufficient for conformity is potentially lower for the third rule and stricter.

Further aspects of the invention will be realized and attained by means of the elements and combinations particularly defined in the dependent claims. It is to be understood that both, the foregoing general description and the following detailed description are exemplary and explanatory only and are not restrictive of the invention which is defined by the appended claims.

**Brief Description of the Drawings**

FIG. 1 includes a block diagram with an example embodiment of a computer system for determining conformity of a fingerprint of a sample with at least one corresponding expected fingerprint of said sample;
FIG. 2 is a simplified flowchart of a computer-implemented method for determining conformity of a fingerprint of a sample with at least one corresponding expected fingerprint of said sample according to an embodiment;
FIG. 3 shows examples of measured sample spectra of in comparison to respective reference spectra;
FIGs. 4A, 4B show examples of conformity tests for process control of production batches;
FIG. 5 shows an example of a conformity test for a mixing process with two components;
FIG. 6 illustrates reference spectra added to a spectrum library for batch control conformity tests;
FIG. 7 illustrates examples of wavelet types of the common Daubechies wavelet family;
FIG. 8 illustrates dilatation change and translation of a Daubechies 4 wavelet over three low frequency wavelet bands;
FIGs. 9A, 9B illustrate a wavelet transformation to several bands (levels) with wavelet coefficients for a spectrum combining two absorbance bands, a baseline and noise; and
FIG. 10 is a diagram that shows an example of a generic computer device and a generic mobile computer device which may be used with the techniques described herein.

**Detailed Description**

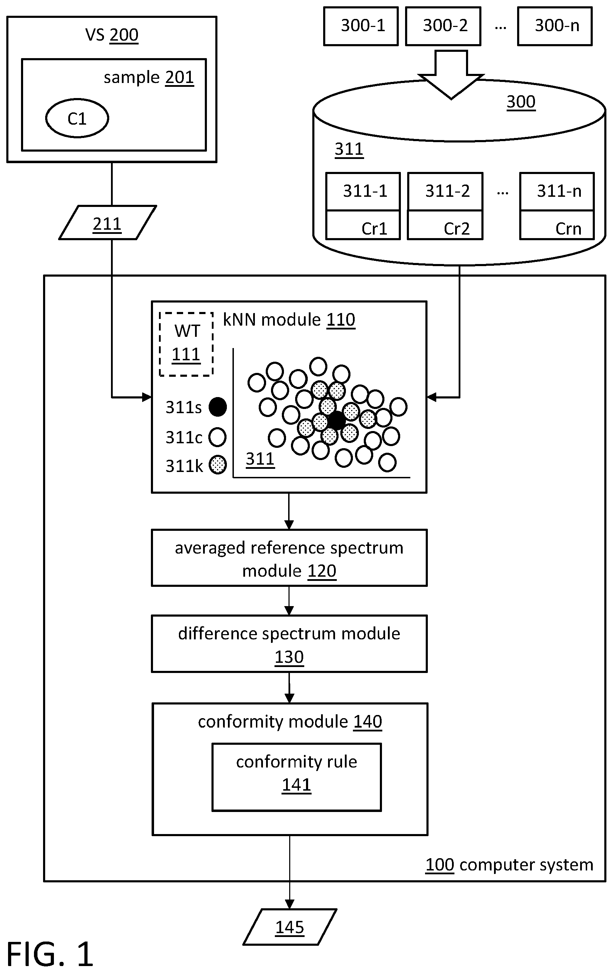

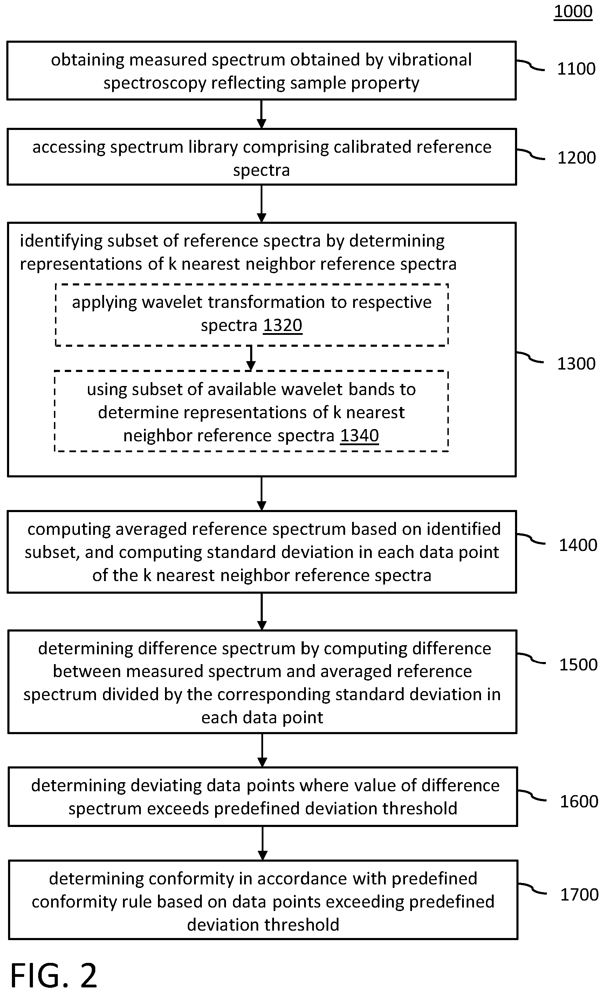

FIG. 1 illustrates a block diagram of an example embodiment of a computer system 100 for determining conformity of a fingerprint of a sample 201 with at least one corresponding expected fingerprint of said sample. FIG. 2 is a simplified flowchart of a computer-implemented method 1000 for determining conformity of a fingerprint of a sample with at least one corresponding expected fingerprint of said sample. Method 1000 can be executed by computer system 100. Therefore, FIG. 1 will be described in the context of FIG. 2 using the respective reference numbers.

The computer system 100 is communicatively coupled to a vibrational spectrometer VS 200. VS 200 can be implemented as a near-infrared (NIR), mid-infrared (MIR) or vibrational Raman spectrometer. VS 200 performs a measurement of said sample 201 resulting in the measured spectrum 211 (NIR-, MIR-, or Raman spectrum). The measured spectrum 211 corresponds to a fingerprint of the sample 201 which reflects the current chemical and physical state C1 of the sample. System 100 obtains 1100 the measured spectrum 211 via an appropriate interface from VS 200. This includes an implementation where the measured spectrum is buffered in an intermediate storage device (not shown) and the system 100 retrieves spectrum 211 from the intermediate storage device. Interfaces between measuring equipment, storage devices and computer systems for data exchange of measurement values are known by a person skilled in the art.

System 100 is further communicatively coupled with a spectrum library 300 comprising a plurality 311 of calibrated reference spectra 311-1 to 311-n. The spectrum library 300 may include multiple reference spectra associated with different states of the sample during various stages of a production process (e.g., a chemical process). Further, the library 300 may include reference spectra for entirely different products (e.g., a various sets of reference spectra for various baking mixtures, various sets of reference spectra for various types of sugar, various sets of reference spectra for milk of different origins, etc.). In a basic implementation, the library 300 may include only a set of reference spectra related to a single process state or to a single product. But even for such a basic implementation, the number of reference spectra will continuously increase over time to keep the library up-to-date, because even for a single product there are always product variants over time which are caused by variations in the raw materials, regional origin, suppliers, seasonal variations, etc. The reference spectra 311-1 to 311-n are obtained by measurements of corresponding reference samples 300-1 to 300-n. Thereby, each reference spectrum can be seen as a corresponding fingerprint for the respective reference sample reflecting the chemical and physical state Cr1 to Crn of the respective reference sample 300-1 to 300-n at the time the measurement was performed to obtain the reference spectrum 311-1 to 311-n.

Once system 100 has obtained the measured spectrum 211, it accesses 1200 the library 300 to identify 1300 a subset of the reference spectra in the spectrum library 300. The to-be-identified subset should include reference spectra in the library 300 which are suitable to evaluate the conformity of the sample 201 with matching reference samples. A matching reference sample is a reference sample where the chemical and physical state of the reference sample has a similarity with the chemical and physical state C1 of the measured sample 201. The system 100 includes a kNN module 110 which is adapted to identify 1300 the subset by determining representations 311k of k nearest neighbor reference spectra in the vicinity of a corresponding representation 311s of the measured spectrum 211 in accordance with predefined metrics. The black bullet 311s represents the measured spectrum in a multidimensional space where the plurality 311 of reference spectra is represented by the circles 311c, 311k.

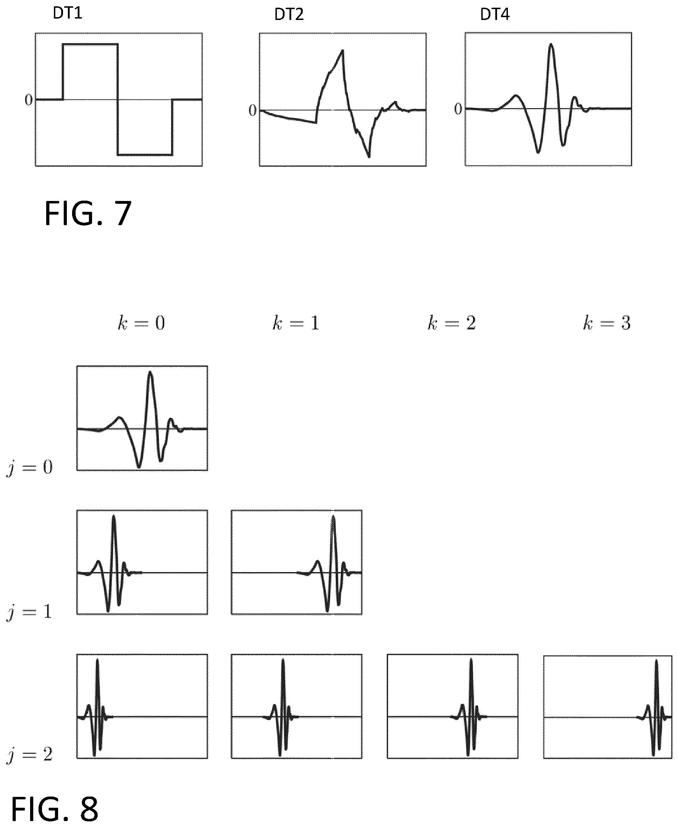

For example, if the representation of the spectra corresponds to the original spectra, a distance between the measured spectrum and a reference spectrum can be determined for each data point of the spectra resulting in a distance vector which has the number of data points as the number of dimensions. Typically, a Minkowski distance metrics is used to determine the k nearest neighbor reference spectra 311k for the measured spectrum 311s. In an optional embodiment, the kNN search is performed in the wavelet space. In this embodiment, firstly a wavelet transformation is applied 1320 to the respective spectra, and secondly, a subset of available wavelet bands is used 1340 to determine representations of k nearest neighbor reference spectra in the library 300. This optional embodiment is described in more detail in the context of FIGs. 7 to 9B.

An advantage of using a kNN search for the identification of the subset is that the system automatically identifies the suitable reference spectra in the library 300 without requiring any input with regard to the sample type of sample 211. That is, even for large libraries comprising reference spectra for many different products and/or process states, the system 100 autonomously identifies appropriate reference spectra suitable for the following conformity test.

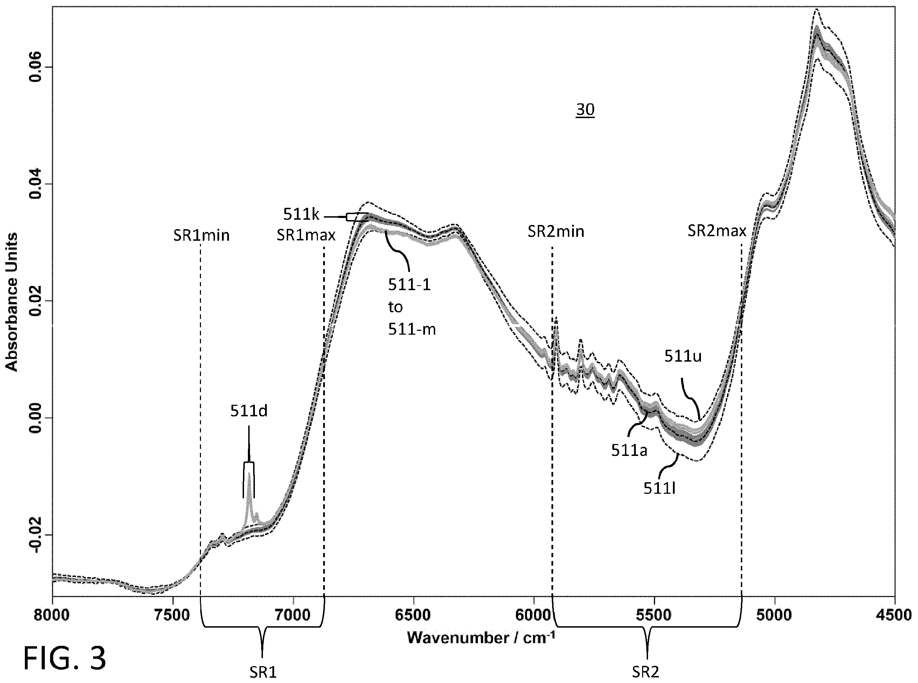

For the conformity test, an averaged reference spectrum module 120 computes 1400 an averaged reference spectrum 511a based on the identified subset 311k. Further, it computes the standard deviation in each data point of the k nearest neighbor reference spectra. Turning briefly to FIG. 3, plot 30 illustrates an example result of the computing step 1400. In FIG. 3, the dark grey reference spectra 511k correspond to the subset of reference spectra identified by the kNN search. The averaged reference spectrum is shown as the dashed line 511a. The standard deviation in each data point is illustrated by the dashed lines 511l, 511u. Thereby, the lower and upper bounds 511l, 511u define a band which provides the basis for the conformity test of the measured spectrum. If a measured spectrum is within the boundaries of [511l, 511u] for all wavenumbers, then conformity is of course given. However, there are also less strict conformity rules which will be discussed in the following.

In FIG. 3, a plurality of light grey lines 511-1 to 511-m represents 10 measured spectra which are to be assessed with regard to their conformity with the identified subset 511k. To finally assess the conformity of measured spectra, system 100 uses a difference spectrum module 130 to determine 1500 a difference spectrum by computing the difference between a measured spectrum and the averaged reference spectrum divided by the corresponding standard deviation in each data point. In other words, for each wavenumber i the absolute difference between the measured spectrum and the averaged reference spectrum is calculated. This absolute difference is weighted by the corresponding standard deviation $\sigma_i$ at that wavenumber. This allows the system to determine 1600 deviating data points where the value of the difference spectrum exceeds a predefined deviation threshold. Advantageously, the predefined deviation threshold is in the range from 3 to 8 (based on the respective standard deviations). The result of the computation in the determining 1500 step for the calculation of the relative deviation for each wavenumber i of a particular measured spectrum is also referred to as conformity index CI as defined in formula F1:

$$CI = \frac{A_{reference,i} - A_{sample,i}}{\sigma_{reference,i}} \qquad (F1)$$

with $A_{reference,i}$ being the absorbance value of the averaged reference spectrum an $A_{sample,i}$ being the absorbance value of the measured spectrum at wavenumber i. The standard deviation is computed based on the reference spectra. The absolute difference between measured sample spectrum and averaged reference spectrum is then divided by the standard deviation. This results in a dimensionless value - the conformity index - being compared to the predefined deviation threshold, which advantageously is in the range from 3 to 8.

In the conformity test, one data point exceeding the threshold would cause, as a result, that the sample is not conform. To simplify the results of checking, for a plurality of data points in the sample spectrum, a maximum conformity index (MCI) can be derived from the results. Thereby, the data point with the maximum MCI value is sufficient to be checked against the threshold, and in addition to the conformity test result (conform or not), the MCI can be provided as additional information result (e.g., in plotted format).

In the example of FIG. 3, the measured spectra 511-1 to 511-m show a significant deviation from the averaged reference spectrum 511a for wavenumbers around the peak at about wavenumber 7200. The wavenumber interval 511d indicates the wavenumbers with deviating data points for a predefined threshold for the conformity index CI. In the example, the conformity test also would provide a correct result when using only sub-range SR1 defined by [SR1min, SR1max] as the measured spectrum in combination with the respective sub-range SR1 for the reference spectra. The further sub-range SR2 defined by [SR2min, SR2max] can also be a part of the measured spectrum used for the conformity test. However, in the example of FIG. 3, no deviating data points are detected in SR2. In other words, the conformity test can define the spectral range (e.g., the entire spectrum or just one or more sub-ranges SR1, SR2), which are to be used for the evaluation of the spectra to assess conformity of the measured spectrum with the kNN reference spectra.

Turning back to FIG. 1, system 100 further has a conformity module 140 which is adapted to determine 1700 conformity 145 in accordance with a predefined conformity rule 141 based on the data points exceeding the predefined deviation threshold. Different conformity rules can be implemented by the conformity module 140:

- a first conformity rule determines conformity if no deviating data point is determined for the entire measured spectrum. That is, the value of the difference spectrum does not exceed the predefined deviation threshold over the entire spectrum.

- a second conformity rule determines conformity if the average value of the values of the difference spectrum at the deviating data points is below a predefined further threshold. That is, the average of the values for deviating data points is compared to the predefined further threshold. The predefined further threshold may be in the range from 0.01 to 2 (based on the standard deviations). Advantageously, it is in the range from 0.01 to 0.5.

- a third conformity rule determines conformity if the sum of the values of the difference spectrum at the deviating data points divided by the total number of all data points is below the predefined further threshold. In this implementation, the sum of the deviations is set in relation to all data points of the spectrum before being compared with the predefined further threshold.

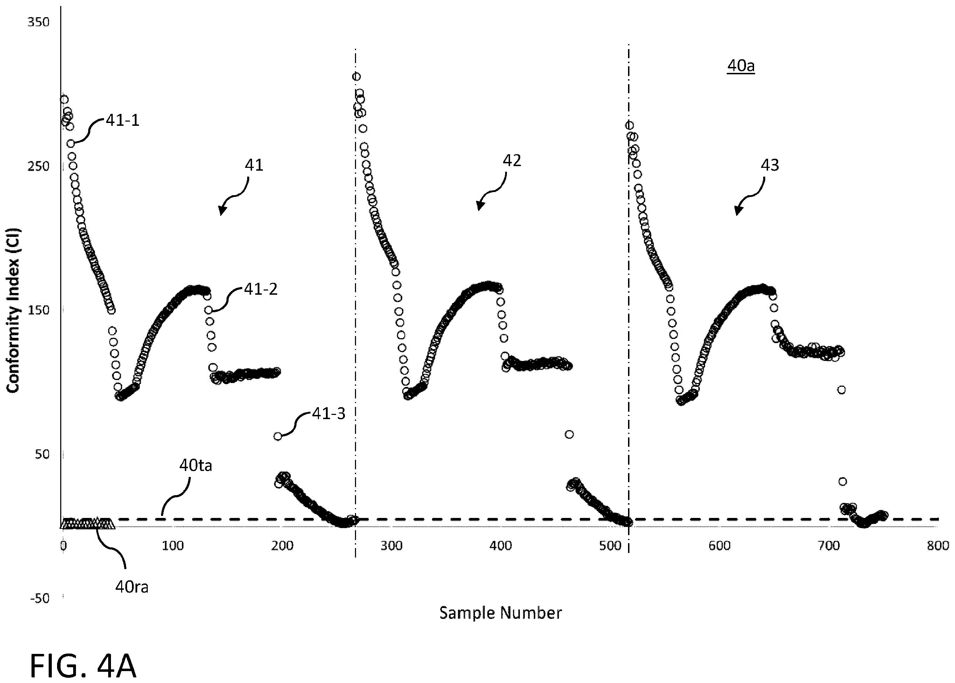

FIG. 4A shows an example of a conformity test 40a for process control with three batches 41, 42, 43 (separated by dash-dotted lines). The three batches are consecutively run. Vibrational spectroscopy measurements are performed at regular intervals to obtain a respective measured spectrum. The current chemical and physical state associated with the measured spectrum at each measurement defines a corresponding sample at the time of the measurement. Therefore, the sample number axis reflects a time line for the processing of the samples. Each sample is represented by a circle. For example, in batch 41, sample 41-1 is measured during the initial phase of the monitored process, sample 41-2 is measured during the middle phase, and sample 41-3 is measured at the beginning of the final phase.

The triangles 40ra at the lower left corner of the diagram represent reference sample spectra which were used to determine the average reference spectrum for the final end product - i.e., the product at the end of each batch 41, 42, 43. The corresponding predefined deviation threshold (e.g., 3) is shown as dashed line 40ta. That is, data points with a conformity index CI greater than the predefined deviation threshold 40t are considered to be deviating data points. The goal of the process is to produce a product with a fingerprint that complies with the conformity test in relation to the reference samples. Each batch starts with measured spectra which are far away from the target (reference) spectra. During the initial phase of each batch, the samples change rapidly to a state with CI values approaching the threshold 40t but still having to large CI values to be considered as conform. During the middle phase of each batch, some reaction occurs in the process and the conformity index even moves further away from the threshold, before, in the last process phase, a continuous approaching to the target state of the sample is achieved. In batches 41 and 42 the final sample has a conformity index which is below the predefined deviation threshold. In batch 43, the conformity index of the final sample is slightly above the predefined deviation threshold. Nevertheless, dependent on the applied conformity rule and a respective predefined further threshold, conformity of the final sample with the respective reference samples can be confirmed for all three batches.

For the conformity check of the final sample fingerprint with the reference sample fingerprints a relatively low number of reference spectra can be sufficient, However, in case the entire process is to be monitored, the reference spectrum library will include a plurality of reference spectra for each point in time where a sample measurement is performed. That is, for each intermediate chemical and physical state of the sample, a plurality of reference spectra is available in the library. To check that the process is being executed as expected, the herein disclosed approach is able to automatically identify the relevant subset of reference spectra for each measured spectrum by using the kNN search, and then apply the conformity test to the measured spectrum in relation to the identified subset of relevant reference spectra.

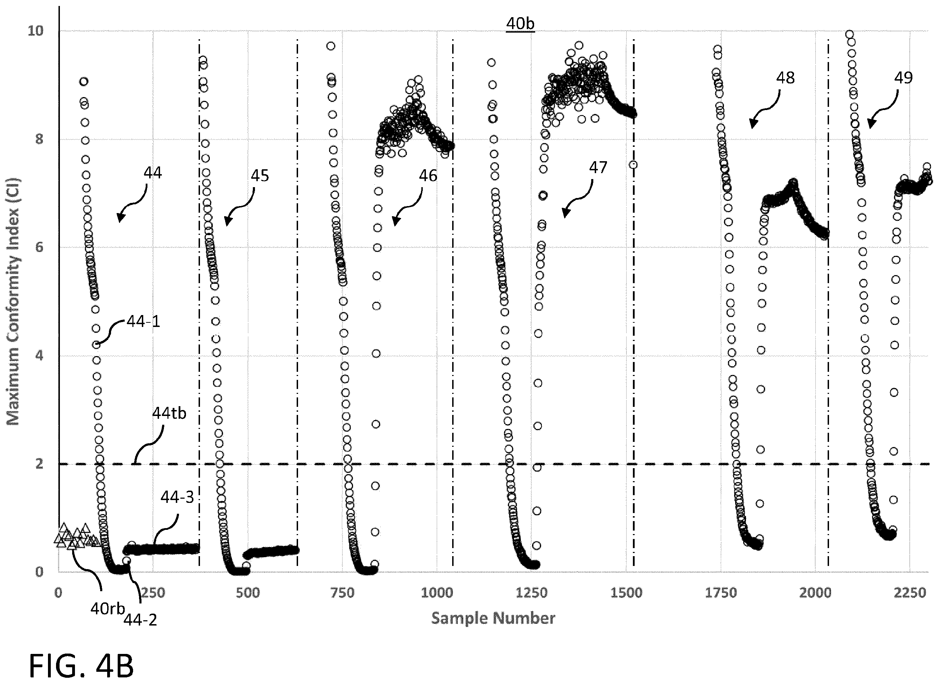

FIG. 4B illustrates a second batch control example scenario with six batches 44 to 49. Here, the maximum conformity index is shown for various sample states (illustrated by circles) during the batch processes. The maximum conformity index (MCI) reflects the result at the data point with maximum deviation of the measured spectrum from the average reference spectrum over a plurality of data points (e.g., the entire spectrum). In the example, conformity is confirmed if the MCI is below the predefined deviation threshold. In other words, if the MCI is below the corresponding deviation threshold factor expressed as (fractional) multiples of the standard deviation, conformity is confirmed. The reference spectra for the final end product are again illustrated as triangles 40rb. The predefined deviation threshold 44tb in this is example is less strict with regard to the final end product than in FIG. 4A. The first wo batches 44, 45 reflect expected process flows where, in an initial phase, the MCI continuously decreases until a minimum is reached. Sample 44-1 is part of this initial phase. During a mid-phase, the MCI increases again up to the level of the reference spectra 40rb. Sample 44-2 is part of the mid-phase. In the final phase, the MCI stays relatively stable at the value which corresponds to the expected end product. The batch behavior of batch 45 is relatively similar to batch 44. However, during the final phase of the batch a slight continuous increase up to the value for the expected end product can be observed.

The behavior of batches 46 to 49 clearly deviates from the expected batch behavior. The initial phases of these batches run according to the expectations. However, during the mid-phase the batches do not approach an MCI value which is already close to the final expected value. Rather, the MCI values increase again substantially going far beyond the predefined deviation threshold. In batches 46 and 47, the final phase appears to be substantially uncontrolled which is reflected by the high variations of the samples during the final phase. When the final phase stabilized, the MCI value of the final product is about four times the predefined deviation threshold value '2'. Although in batches 48, 49 the final phase shows less variations than in batches 46, 47, still the MCI value of the final product is more than three times higher than the predefined deviation threshold value '2'. As a conclusion, the batches 46 to 49 do not lead to a final product which corresponds to the expected product. The reason appears to an issue which occurs during the mid-phase of the batches when all of sudden the MCI value substantially exceeds the predefined deviation threshold.

For example, the production system for running the batches can implement an automatic stop function for a batch once the conformity test during the mid-phase provides MCI values for the samples which exceed the predefined deviation threshold 44tb. Stopping the batch shortly after the mid-phase crossing of the predefined deviation threshold 44tb can save energy and production resources by nor further processing samples which in the end will not lead to a final product passing the conformity test.

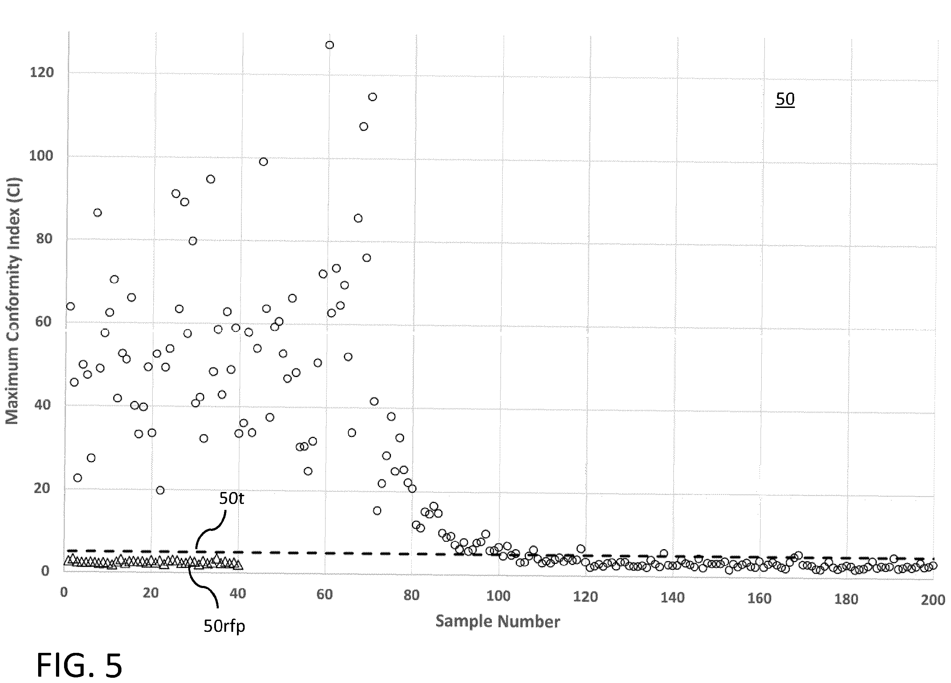

FIG. 5 shows an example of a conformity test 50 for a mixing process where two different white powders (starch and calcium carbonate) were mixed. Again, the sample number axis reflects the time line of the mixing process as the measured spectra for the samples (represented by circles) of the mixed powders were taken at regular time intervals. 40 reference spectra (illustrated by the triangles 50rtp in the lower left of the chart) were measured for reference samples with a homogenous mixture of the two powders. The reference samples reflect the target state of the sample at the end of the mixing process. The strong variations in the conformity index during the initial 80 measurements indicate an inhomogeneous mixture during this early phase of the mixing process. A probe was used for the measurement with a measuring spot of about 3 mm in diameter. Within this measuring spot, the number of powder particles is in the range of 200. The current mixing ratio of the sample impacts the conformity index for the respective sample number. That is, during the first 80 measurements, the mixing ratio still varied significantly from sample to sample. After 90 measurements, the mixture started to become homogeneous and after 110 measurements almost all measurements resulted in a conformity index below the predefined deviation threshold 50t. From a product perspective, the conformity test 50 allows to test whether the targeted product quality for the powder mixture is achieved at the end of the mixing process. Further, from a process perspective, the test also provides the information, when the targeted quality is achieved. This information can be used by the system as input for dynamically generating process control instructions. For example, the mixing process could have been stopped by the system already after measurement 110 for saving energy. In general, using the conformity index for controlling the length of a process allows to run the process just as long as needed to achieve the target product quality, thus saving time, energy and machine resources.

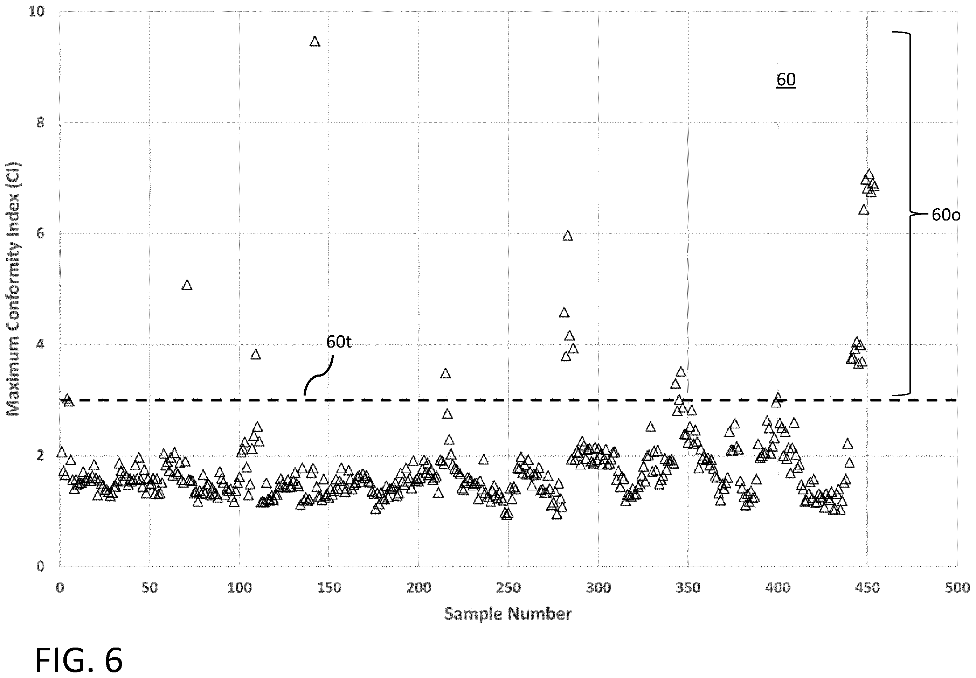

FIG. 6 illustrates reference spectra (represented by triangles) added to a spectrum library for batch control conformity tests. The reference spectra obtained from more than 450 samples in this example also include some spectra 60o with a conformity index significantly exceeding the predefined deviation threshold 60t. In scenarios where higher variances in product quality are acceptable, measured spectra with higher deviations may nevertheless be included in the reference spectrum library to increase the standard deviation in respective data points. This makes the conformity text more tolerant with regards to such acceptable deviations. For example, only measured spectra which clearly result from faulty measurements may be disregarded as reference spectra. Such faulty measurements can easily be distinguished from measured spectra associated with acceptable product deviations because the conformity index for spectra obtained from such faulty measurements is orders of magnitude larger (e.g., > 10000) than for spectra associated with deviating products.

The data used for FIG. 6 illustrate an example showing that deviating samples are not necessarily a problem when they are part of the reference spectra library. Due to the herein disclosed statistical approach using a standard deviation on each data point, a certain number of deviating spectra are not changing the overall picture immediately. This is advantageous for the dynamic approach where reference spectra are selected via kNN search. As long as the number of selected spectra is sufficiently high (e.g., at least 50), the presence of deviating spectra in the reference spectra does not impose a significant risk. However, finally this depends on the data itself and the resulting statistics.

As mentioned in the description of FIG. 1, in an optional embodiment, the representations of the measured spectrum and the reference spectra in the library are in the wavelet space. Such representations are obtained by applying 1320 a wavelet transformation to the respective spectra. For this purpose, the kNN module may 110 have a wavelet transformation submodule WT 111, which applies the wavelet transformation to the measured spectrum 211 and the references spectra of the library 300. It may be advantageous to already store wavelet transformed reference spectra in the library so that they can be immediately accessed by the kNN module 110. In this implementation, WT 111 only needs to generate a wavelet transformation of the measured spectrum 211. The identifying of the kNN subset is then performed in accordance with predefined metrics in the wavelet space. The predefined metrics in the wavelet space may also be a Minkowski distance.

The wavelet transformation module WT 111 applies a wavelet transformation to the measured spectrum 211 and thereby computes a sample set of wavelet coefficients in a plurality of wavelet bands, for example in three wavelet bands B0, B1, B2. However, typically the number of wavelet bands is higher. In general, a wavelet transformation into any wavelet type can be used. This includes but is not limited to the following wavelet types: Haar wavelet; Daubechies wavelets db2, db4, db6, and db8; Morlets; Symlets; Coiflets; and Biorthogonal wavelets. Other wavelet types may be used, too.

Turning briefly to FIG. 7, three different example wavelet types DT1, DT2 and DT4 of the common Daubechies wavelet family are shown. DT1 illustrates the Daubechies type 1 (often referred to as Haar). DT2 and DT4 illustrate the Daubechies types 2 and 4, respectively. Such simple wavelet transformation types can be used for operating the wavelet transformation because they serve the purpose to decompose the measured spectrum 211 to a representation in the wavelet space for using the computed wavelet coefficients of the sample set directly for the kNN search. In particular when using a discrete wavelet transformation, the wavelet transformation and the coefficients of the sample set are orthogonal. With other types of wavelet transformations, it is still possible to select coefficients which are orthogonal. Optionally, the wavelet coefficients of the sample set may be normalized by using vector normalization.

Turning briefly to FIG. 8, dilation change and translation of a Daubechies 4 wavelet is illustrated over three low frequency wavelet bands j=0, j=1, and j=2. The wavelet of band j=0 can be adapted in width to cover different frequency components in the signal in higher bands. For example, the width of the wavelet is reduced in band j=1 and again in band j=2. A narrow-scaled wavelet can also analyze a higher frequency. By translation of the wavelet over the signal this frequency can be analyzed at different locations of the spectrum. Similarly, the even more narrowed wavelet in band j=2 can be used to analyze a spectrum at four different spectrum locations (k=0 to k=3). For determining representations of k nearest neighbor reference spectra, only a subset of available wavelet bands may be used 1340. Advantageously, this subset of available wavelet bands comprises wavelet bands with low and medium frequencies. Thereby, typically at least the lowest wavelet band and the highest wavelet band of the computed sample set are ignored as they do not contain relevant information because NIR/MIR/Raman signals are almost always reflected in the wavelet bands between such extreme bands. From a user input, the system may receive one or more additional higher bands to be ignored. For example, the system may only use the coefficients of one wavelet band for the nearest neighbor search in the library to keep computation time low. The nearest neighbor search over the kNN slows down exponentially with an increasing number of dimensions. A medium wavelet band can already represent the most important spectral information (with sufficient variance) to identify appropriate kNNs.

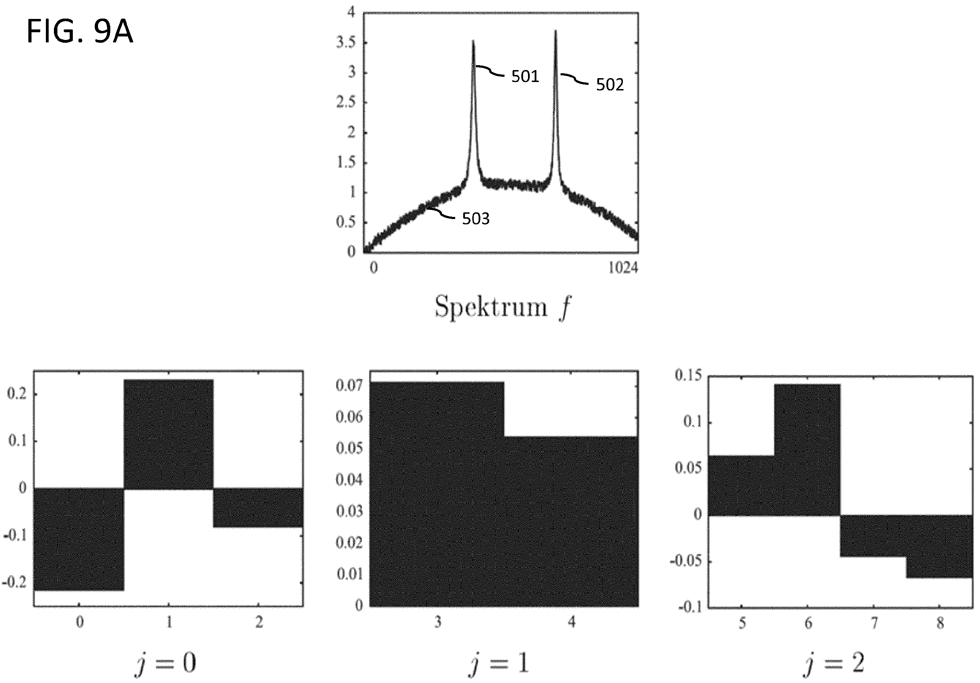

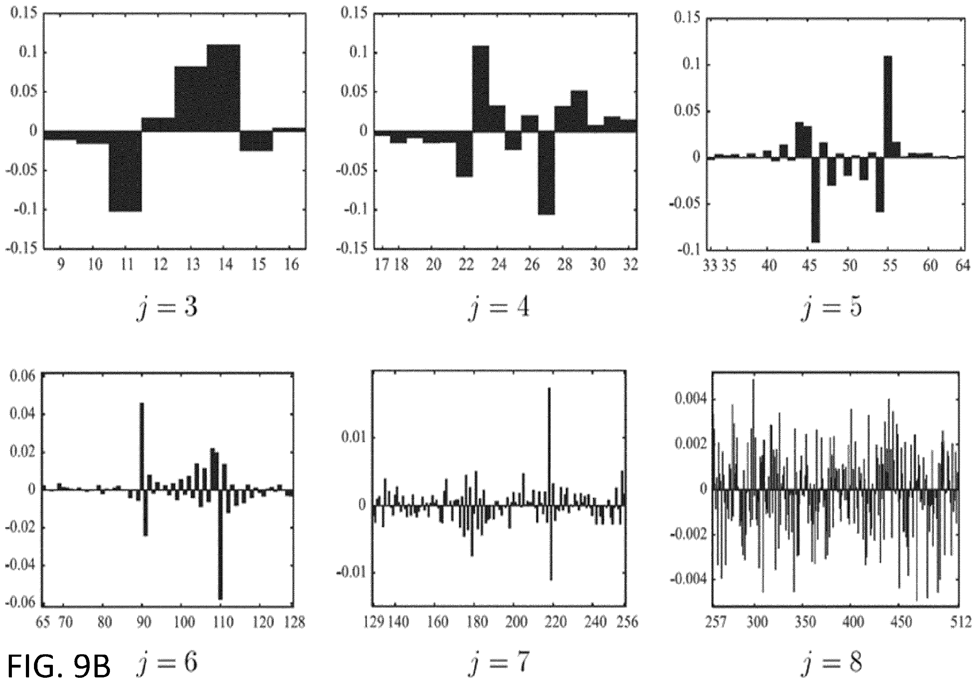

Turning briefly to FIGs. 9A, 9B, the wavelet transformation is further explained by using an example with a spectrum f which includes two absorbance bands 501, 502, a baseline (the bended bow shaped curve 503 extending from 0 to 1024 data points) and some noise which is superimposed to the base line. For ease of explanation, example spectrum f has been obtained by simulation.

The wavelet coefficients of the wavelet transform are sorted in wavelet bands (or levels) starting from a low frequency band (j=1) with only one or two coefficients covering the whole spectral range of the signal (the simulated spectrum f). As mentioned earlier, the coefficients of band j=0 are the so-called approximation or scaling coefficients having no relevance for the herein disclosed method and system and are therefore ignored by the further processing steps. In the example, band j=1 has coefficients 3, 4, band j=2 has coefficients 5 to 8, band j=3 has coefficients 9 to 16, and so on. The number of coefficients is always doubled in the next higher band and the corresponding analyzed frequency is increased. The highest band(s) (j=8, j=7) in the example of FIG. 9B show mainly noise. However, in case of a smooth spectrum, also such highest bands may still include some valuable information. The valuable coefficients are those with values higher than the values representing merely noise. In the simulated spectrum f, one can easily see which coefficients are important or valuable as they contain information about the two absorbance bands. Wavelet bands below band j=3 represent mainly baseline information or less important low frequency information. On the other hand, the higher bands j=7 and j=8 primarily represent noise. That is, the valuable information about the two absorbance peaks in spectrum f is in the bands j=3 to j=6.

The reference spectra 300-1 to 300-n are processed with the same wavelet transformation as the measured spectrum 211 to allow a kNN search in the wavelet space. It can be advantageous to process each reference spectrum already when adding the spectrum to the library 300 and store the wavelet transformed reference spectra together with the original reference spectra in the library. In this implementation, the kNN module 110 can immediately retrieve the wavelet transformed spectra from the library for performing the kNN search and does not need to do the transformation of reference spectra on its own. That is, in this implementation WT 111 only transforms the measured spectrum 211.

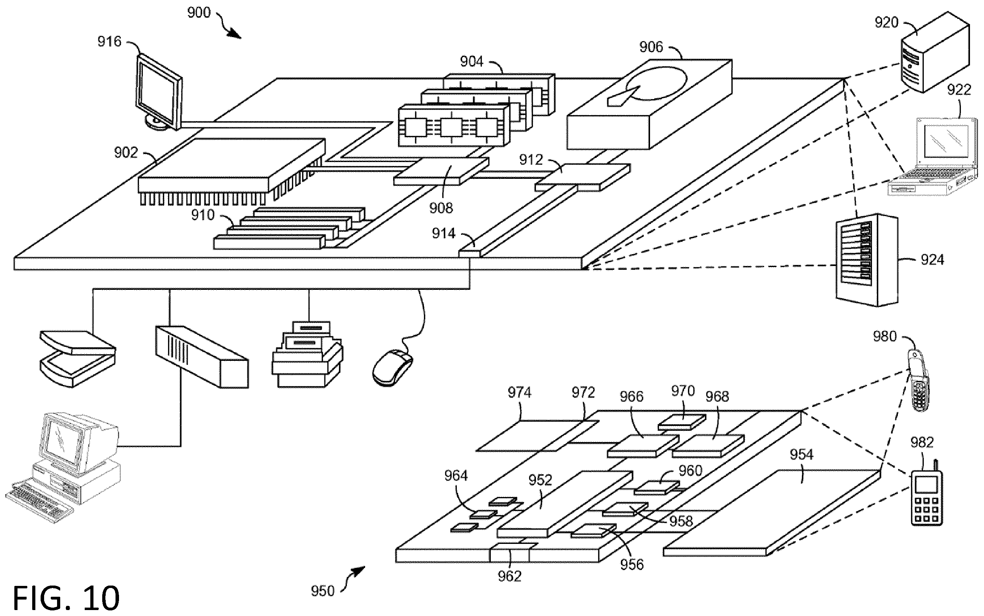

FIG. 10 is a diagram that shows an example of a generic computer device 900 and a generic mobile computer device 950, which may be used with the techniques described here. In some embodiments, computing device 900 may relate to system 100 (cf. FIG. 1). Computing device 950 is intended to represent various forms of mobile devices, such as personal digital assistants, cellular telephones, smart phones, and other similar computing devices. In the context of this disclosure the computing device 950 may provide I/O means for a user to interact with the computing device 900 (e.g., for receiving a selection of wavelet bands). The components shown here, their connections and relationships, and their functions, are meant to be exemplary only, and are not meant to limit implementations of the inventions described and/or claimed in this document.

Computing device 900 includes a processor 902, memory 904, a storage device 906, a high-speed interface 908 connecting to memory 904 and high-speed expansion ports 910, and a low speed interface 912 connecting to low speed bus 914 and storage device 906. Each of the components 902, 904, 906, 908, 910, and 912, are interconnected using various busses, and may be mounted on a common motherboard or in other manners as appropriate. The processor 902 can process instructions for execution within the computing device 900, including instructions stored in the memory 904 or on the storage device 906 to display graphical information for a GUI on an external input/output device, such as display 916 coupled to high speed interface 908. In other implementations, multiple processors and/or multiple buses may be used, as appropriate, along with multiple memories and types of memory. Also, multiple computing devices 900 may be connected, with each device providing portions of the necessary operations (e.g., as a server bank, a group of blade servers, or a multi-processor system).

The memory 904 stores information within the computing device 900. In one implementation, the memory 904 is a volatile memory unit or units. In another implementation, the memory 904 is a non-volatile memory unit or units. The memory 904 may also be another form of computer-readable medium, such as a magnetic or optical disk.

The storage device 906 is capable of providing mass storage for the computing device 900. In one implementation, the storage device 906 may be or contain a computer-readable medium, such as a floppy disk device, a hard disk device, an optical disk device, or a tape device, a flash memory or other similar solid state memory device, or an array of devices, including devices in a storage area network or other configurations. A computer program product can be tangibly embodied in an information carrier. The computer program product may also contain instructions that, when executed, perform one or more methods, such as those described above. The information carrier is a computer- or machine-readable medium, such as the memory 904, the storage device 906, or memory on processor 902.

The high speed controller 908 manages bandwidth-intensive operations for the computing device 900, while the low speed controller 912 manages lower bandwidth-intensive operations. Such allocation of functions is exemplary only. In one implementation, the high-speed controller 908 is coupled to memory 904, display 916 (e.g., through a graphics processor or accelerator), and to high-speed expansion ports 910, which may accept various expansion cards (not shown). In the implementation, low-speed controller 912 is coupled to storage device 906 and low-speed expansion port 914. The low-speed expansion port, which may include various communication ports (e.g., USB, Bluetooth, Ethernet, wireless Ethernet) may be coupled to one or more input/output devices, such as a keyboard, a pointing device, a scanner, or a networking device such as a switch or router, e.g., through a network adapter.

The computing device 900 may be implemented in a number of different forms, as shown in the figure. For example, it may be implemented as a standard server 920, or multiple times in a group of such servers. It may also be implemented as part of a rack server system 924. In addition, it may be implemented in a personal computer such as a laptop computer 922. Alternatively, components from computing device 900 may be combined with other components in a mobile device (not shown), such as device 950. Each of such devices may contain one or more of computing device 900, 950, and an entire system may be made up of multiple computing devices 900, 950 communicating with each other.

Computing device 950 includes a processor 952, memory 964, an input/output device such as a display 954, a communication interface 966, and a transceiver 968, among other components. The device 950 may also be provided with a storage device, such as a microdrive or other device, to provide additional storage. Each of the components 950, 952, 964, 954, 966, and 968, are interconnected using various buses, and several of the components may be mounted on a common motherboard or in other manners as appropriate.

The processor 952 can execute instructions within the computing device 950, including instructions stored in the memory 964. The processor may be implemented as a chipset of chips that include separate and multiple analog and digital processors. The processor may provide, for example, for coordination of the other components of the device 950, such as control of user interfaces, applications run by device 950, and wireless communication by device 950.

Processor 952 may communicate with a user through control interface 958 and display interface 956 coupled to a display 954. The display 954 may be, for example, a TFT LCD (Thin-Film-Transistor Liquid Crystal Display) or an OLED (Organic Light Emitting Diode) display, or other appropriate display technology. The display interface 956 may comprise appropriate circuitry for driving the display 954 to present graphical and other information to a user. The control interface 958 may receive commands from a user and convert them for submission to the processor 952. In addition, an external interface 962 may be provide in communication with processor 952, so as to enable near area communication of device 950 with other devices. External interface 962 may provide, for example, for wired communication in some implementations, or for wireless communication in other implementations, and multiple interfaces may also be used.

The memory 964 stores information within the computing device 950. The memory 964 can be implemented as one or more of a computer-readable medium or media, a volatile memory unit or units, or a non-volatile memory unit or units. Expansion memory 984 may also be provided and connected to device 950 through expansion interface 982, which may include, for example, a SIMM (Single In Line Memory Module) card interface. Such expansion memory 984 may provide extra storage space for device 950, or may also store applications or other information for device 950. Specifically, expansion memory 984 may include instructions to carry out or supplement the processes described above, and may include secure information also. Thus, for example, expansion memory 984 may act as a security module for device 950, and may be programmed with instructions that permit secure use of device 950. In addition, secure applications may be provided via the SIMM cards, along with additional information, such as placing the identifying information on the SIMM card in a non-hackable manner.

The memory may include, for example, flash memory and/or NVRAM memory, as discussed below. In one implementation, a computer program product is tangibly embodied in an information carrier. The computer program product contains instructions that, when executed, perform one or more methods, such as those described above. The information carrier is a computer- or machine-readable medium, such as the memory 964, expansion memory 984, or memory on processor 952, that may be received, for example, over transceiver 968 or external interface 962.

Device 950 may communicate wirelessly through communication interface 966, which may include digital signal processing circuitry where necessary. Communication interface 966 may provide for communications under various modes or protocols, such as GSM voice calls, SMS, EMS, or MMS messaging, CDMA, TDMA, PDC, WCDMA, CDMA2000, or GPRS, among others. Such communication may occur, for example, through radio-frequency transceiver 968. In addition, short-range communication may occur, such as using a Bluetooth, WiFi, or other such transceiver (not shown). In addition, GPS (Global Positioning System) receiver module 980 may provide additional navigation- and location-related wireless data to device 950, which may be used as appropriate by applications running on device 950.

Device 950 may also communicate audibly using audio codec 960, which may receive spoken information from a user and convert it to usable digital information. Audio codec 960 may likewise generate audible sound for a user, such as through a speaker, e.g., in a handset of device 950. Such sound may include sound from voice telephone calls, may include recorded sound (e.g., voice messages, music files, etc.) and may also include sound generated by applications operating on device 950.

The computing device 950 may be implemented in a number of different forms, as shown in the figure. For example, it may be implemented as a cellular telephone 980. It may also be implemented as part of a smart phone 982, personal digital assistant, or other similar mobile device.

Various implementations of the systems and techniques described here can be realized in digital electronic circuitry, integrated circuitry, specially designed ASICs (application specific integrated circuits), computer hardware, firmware, software, and/or combinations thereof. These various implementations can include implementation in one or more computer programs that are executable and/or interpretable on a programmable system including at least one programmable processor, which may be special or general purpose, coupled to receive data and instructions from, and to transmit data and instructions to, a storage system, at least one input device, and at least one output device.

These computer programs (also known as programs, software, software applications or code) include machine instructions for a programmable processor, and can be implemented in a high-level procedural and/or object-oriented programming language, and/or in assembly/machine language. As used herein, the terms "machine-readable medium" "computer-readable medium" refers to any computer program product, apparatus and/or device (e.g., magnetic discs, optical disks, memory, Programmable Logic Devices (PLDs)) used to provide machine instructions and/or data to a programmable processor, including a machine-readable medium that receives machine instructions as a machine-readable signal. The term "machine-readable signal" refers to any signal used to provide machine instructions and/or data to a programmable processor.

To provide for interaction with a user, the systems and techniques described here can be implemented on a computer having a display device (e.g., a CRT (cathode ray tube) or LCD (liquid crystal display) monitor) for displaying information to the user and a keyboard and a pointing device (e.g., a mouse or a trackball) by which the user can provide input to the computer. Other kinds of devices can be used to provide for interaction with a user as well; for example, feedback provided to the user can be any form of sensory feedback (e.g., visual feedback, auditory feedback, or tactile feedback); and input from the user can be received in any form, including acoustic, speech, or tactile input.

The systems and techniques described here can be implemented in a computing device that includes a back end component (e.g., as a data server), or that includes a middleware component (e.g., an application server), or that includes a front end component (e.g., a client computer having a graphical user interface or a Web browser through which a user can interact with an implementation of the systems and techniques described here), or any combination of such back end, middleware, or front end components. The components of the system can be interconnected by any form or medium of digital data communication (e.g., a communication network). Examples of communication networks include a local area network ("LAN"), a wide area network ("WAN"), and the Internet.

The computing device can include clients and servers. A client and server are generally remote from each other and typically interact through a communication network. The relationship of client and server arises by virtue of computer programs running on the respective computers and having a client-server relationship to each other.

**Claims**

1. A computer-implemented method (1000) for determining conformity of a fingerprint of a sample (201) with a corresponding expected fingerprint of said sample, comprising:

   obtaining (1100) a measured spectrum (211) as the fingerprint of the sample (201), wherein the measured spectrum is the result of a measurement reflecting a current chemical and physical state (C1) of the sample obtained by vibrational spectroscopy;
   **characterized in that**
   accessing (1200) a spectrum library (300) comprising a plurality (311) of calibrated reference spectra (311-1 to 311-n);
   identifying (1300) a subset of the reference spectra in the spectrum library, wherein the subset is identified by determining representations (311k) of k nearest neighbor reference spectra in the vicinity of a corresponding representation (311s) of the measured spectrum (211) in accordance with predefined metrics;
   computing (1400), based on the identified subset, an averaged reference spectrum as the corresponding expected fingerprint, and computing the standard deviation in each data point of the k nearest neighbor reference spectra;
   determining (1500) a difference spectrum by computing the difference between measured spectrum and averaged reference spectrum divided by the corresponding standard deviation in each data point;
   determining (1600) deviating data points where the value of the difference spectrum exceeds a predefined deviation threshold; and
   determining (1700) conformity (145) in accordance with a predefined conformity rule (141) based on the data points exceeding said predefined deviation threshold.

2. The method of claim 1, wherein the predefined deviation threshold is in the range from 3 to 8.

3. The method of claim 1 or 2, wherein the conformity rule determines conformity if no deviating data point is determined.

4. The method of claim 1 or 2, wherein the conformity rule determines conformity if the average value of the values of the difference spectrum at the deviating data points is below a predefined further threshold.

5. The method of claim 1 or 2, the conformity rule determines conformity if the sum of the values of the difference spectrum at the deviating data points divided by the total number of all data points is below a predefined further threshold.

6. The method of claim 4 or 5, wherein the predefined further threshold is in the range from 0.01 to 2, preferably 0.01 to 0.5.

7. The method of any of the previous claims, wherein the representations of the measured spectrum and the reference spectra in the library are obtained by applying (1320) a wavelet transformation to the respective spectra, and wherein the identifying of the subset is performed in accordance with predefined metrics in the wavelet space.

8. The method of claim 7, wherein determining representations of k nearest neighbor reference spectra uses (1340) a subset of available wavelet bands.

9. The method of claim 8, wherein the subset of available wavelet bands comprises wavelet bands with low and medium frequencies wherein the subset only includes wavelet bands between the lowest wavelet band and the highest wavelet band.

10. The method of any of the previous claims, wherein the predefined metrics is a Minkowski distance.

11. The method of any of the previous claims, wherein the fingerprint is characteristic of the sample in any of the following aspects: the quality of the sample, the composition of the sample.

12. The method of any of the previous claims, wherein the measured spectrum and the reference spectra are obtained either by using infrared spectroscopy or by using Raman spectroscopy.

13. The method of any of the previous claims, wherein the spectrum library comprises reference spectra for a plurality of different expected characteristic states of a sample which occur during the processing of the sample, each characteristic state being associated with a respective fingerprint, and wherein, for each sub-process of the processing, an appropriate subset of respective reference spectra is identified via a respective k nearest neighbor search.

14. A computer program product for determining conformity of a fingerprint of a sample with a corresponding expected fingerprint of said sample, comprising computer-readable instructions that, when loaded into the memory of a computing device and processed by one or more processors of the computing device, cause the computing device to execute the method steps according to any of the previous claims.

15. A computer system (100) for determining conformity of a fingerprint of a sample (201) with a corresponding expected fingerprint of said sample, comprising functional modules adapted to execute the method steps according to any of the claims 1 to 13.

**Patentansprüche**

1. Computerimplementiertes Verfahren (1000) zum Bestimmen einer Konformität eines Fingerabdrucks einer Probe (201) mit einem entsprechenden erwarteten Fingerabdruck der Probe, umfassend:

   Erlangen (1100) eines gemessenen Spektrums (211) als Fingerabdruck der Probe (201), wobei das gemessene Spektrum das Ergebnis einer Messung ist, die den aktuellen chemischen und physikalischen Zustand (C1) der Probe widerspiegelt, der durch Schwingungsspektroskopie erlangt wurde;
   **gekennzeichnet durch**:

   Zugreifen (1200) auf eine Spektrenbibliothek {300), umfassend eine Vielzahl (311) kalibrierter Referenzspektren (311-1 bis 311-n);
   Identifizieren (1300) einer Teilmenge der Referenzspektren in der Spektrenbibliothek, wobei die Teilmenge durch Bestimmen von Darstellungen (311k) der k nächsten Nachbarreferenzspektren in der Nähe einer entsprechenden Darstellung (311s) des gemessenen Spektrums (211) gemäß vordefinierten Metriken identifiziert wird;
   Berechnen (1400) eines gemittelten Referenzspektrums als entsprechender erwarteter Fingerabdruck auf Grundlage der identifizierten Teilmenge und Berechnen der Standardabweichung in jedem Datenpunkt der k nächsten Nachbarreferenzspektren;
   Bestimmen (1500) eines Differenzspektrums durch Berechnen der Differenz zwischen dem gemessenen Spektrum und dem gemittelten Referenzspektrum, geteilt durch die entsprechende Standardabweichung in jedem Datenpunkt;
   Bestimmen (1600) von abweichenden Datenpunkten, bei denen der Wert des Differenzspektrums einen vordefinierten Abweichungsschwellenwert überschreitet; und
   Bestimmen (1700) der Konformität (145) nach einer vordefinierten Konformitätsregel (141) basierend auf den Datenpunkten, die den vordefinierten Abweichungsschwellenwert überschreiten.

2. Verfahren nach Anspruch 1, wobei der vordefinierte Abweichungsschwellenwert im Bereich von 3 bis 8 liegt.

3. Verfahren nach Anspruch 1 oder 2, wobei die Konformitätsregel Konformität bestimmt, wenn kein abweichender Datenpunkt bestimmt wird.

4. Verfahren nach Anspruch 1 oder 2, wobei die Konformitätsregel Konformität bestimmt, wenn der Durchschnittswert der Werte des Differenzspektrums an den abweichenden Datenpunkten unter einem vordefinierten weiteren Schwellenwert liegt.

5. Verfahren nach Anspruch 1 oder 2, wobei die Konformitätsregel Konformität bestimmt, wenn die Summe der Werte des Differenzspektrums an den abweichenden Datenpunkten geteilt durch die Gesamtzahl aller Datenpunkte unter einem vordefinierten weiteren Schwellenwert liegt.

6. Verfahren nach Anspruch 4 oder 5, wobei der vordefinierte weitere Schwellenwert im Bereich von 0,01 bis 2, vorzugsweise 0,01 bis 0,5, liegt.

7. Verfahren nach einem der vorherigen Ansprüche, wobei die Darstellungen des gemessenen Spektrums und der Referenzspektren in der Bibliothek durch Anwenden (1320) einer Wavelet-Transformation auf die jeweiligen Spektren erlangt werden und wobei das Identifizieren der Teilmenge gemäß vordefinierten Metriken im Wavelet-Raum durchgeführt wird.

8. Verfahren nach Anspruch 7, wobei zum Bestimmen von Darstellungen von k nächsten Nachbarreferenzspektren eine Teilmenge verfügbarer Wavelet-Bänder verwendet wird (1340).

9. Verfahren nach Anspruch 8, wobei die Teilmenge verfügbarer Wavelet-Bänder Wavelet-Bänder mit niedrigen und mittleren Frequenzen umfasst, wobei die Teilmenge nur Wavelet-Bänder zwischen dem niedrigsten Wavelet-Band und dem höchsten Wavelet-Band beinhaltet.

10. Verfahren nach einem der vorherigen Ansprüche, wobei die vordefinierte Metrik eine Minkowski-Distanz ist.

11. Verfahren nach einem der vorherigen Ansprüche, wobei der Fingerabdruck in einem der folgenden Aspekte charakteristisch für die Probe ist: der Qualität der Probe, der Zusammensetzung der Probe.

12. Verfahren nach einem der vorherigen Ansprüche, wobei das gemessene Spektrum und die Referenzspektren entweder durch Infrarotspektroskopie oder durch Raman-Spektroskopie erlangt werden.

13. Verfahren nach einem der vorherigen Ansprüche, wobei die Spektrenbibliothek Referenzspektren für eine Vielzahl verschiedener erwarteter charakteristischer Zustände einer Probe umfasst, die während der Verarbeitung der Probe auftreten, wobei jeder charakteristische Zustand einem entsprechenden Fingerabdruck zugeordnet ist, und wobei für jeden Teilprozess der Verarbeitung eine geeignete Teilmenge der jeweiligen Referenzspektren über eine Suche nach einem entsprechenden k nächsten Nachbarn identifiziert wird.

14. Computerprogrammprodukt zum Bestimmen der Konformität eines Fingerabdrucks einer Probe mit einem entsprechenden erwarteten Fingerabdruck der Probe, umfassend computerlesbare Anweisungen, die, wenn sie in den Speicher einer Rechenvorrichtung geladen und durch einen oder mehreren Prozessoren der Rechenvorrichtung verarbeitet werden, die Rechenvorrichtung veranlassen, die Verfahrensschritte gemäß einem der vorherigen Ansprüche auszuführen.

15. Computersystem (100) zum Bestimmen der Konformität eines Fingerabdrucks einer Probe (201) mit einem entsprechenden erwarteten Fingerabdruck der Probe, umfassend Funktionsmodule, die dazu ausgelegt sind, die Verfahrensschritte gemäß einem der Ansprüche 1 bis 13 auszuführen.


**Revendications**

1. Procédé mis en œuvre par ordinateur (1000) pour déterminer la conformité d'une empreinte d'un échantillon (201) avec une empreinte attendue correspondante dudit échantillon, comprenant :

l'obtention (1100) d'un spectre mesuré (211) servant d'empreinte de l'échantillon (201), le spectre mesuré étant le résultat d'une mesure reflétant un état chimique et physique (C1) actuel de l'échantillon obtenu par spectroscopie vibrationnelle ;
**caractérisé en ce qu'**il comprend :

l'accès (1200) à une bibliothèque de spectres (300) comprenant une pluralité (311) de spectres de référence étalonnés (311-1 à 311-n) ;
l'identification (1300) d'un sous-ensemble des spectres de référence dans la bibliothèque de spectres, le sous-ensemble étant identifié par détermination de représentations (311k) de k spectres de référence plus proches voisins dans le voisinage d'une représentation correspondante (311s) du spectre mesuré (211) en fonction de mesures prédéfinies ;
le calcul (1400), sur la base du sous-ensemble identifié, d'un spectre de référence moyenné servant d'empreinte attendue correspondante, et le calcul de l'écart-type associé à chaque point de données des k spectres de référence plus proches voisins ;
la détermination (1500) d'un spectre de différence par calcul de la différence entre le spectre mesuré et le spectre de référence moyenné divisée par l'écart-type correspondant associé à chaque point de données ;

la détermination (1600) de points de données déviants dont la valeur du spectre de différence dépasse un seuil d'écart prédéfini ; et

la détermination (1700) de la conformité (145) selon une règle de conformité (141) prédéfinie basée sur les points de données dépassant ledit seuil d'écart prédéfini.

2. Procédé selon la revendication 1, dans lequel le seuil d'écart prédéfini se situe dans une plage allant de 3 à 8.

3. Procédé selon la revendication 1 ou 2, dans lequel la règle de conformité détermine la conformité si aucun point de données déviant n'est déterminé.

4. Procédé selon la revendication 1 ou 2, dans lequel la règle de conformité détermine la conformité si la valeur moyenne des valeurs du spectre de différence aux points de données déviants est inférieure à un seuil supplémentaire prédéfini.

5. Procédé selon la revendication 1 ou 2, dans lequel la règle de conformité détermine la conformité si la somme des valeurs du spectre de différence aux points de données déviants divisée par le nombre total de points de données est inférieure à un seuil supplémentaire prédéfini.

6. Procédé selon la revendication 4 ou 5, dans lequel le seuil supplémentaire prédéfini se situe dans une plage allant de 0,01 à 2, de préférence de 0,01 à 0,5.

7. Procédé selon l'une quelconque des revendications précédentes, dans lequel les représentations du spectre mesuré et des spectres de référence dans la bibliothèque sont obtenues par application (1320) d'une transformation d'ondelettes aux spectres respectifs, et dans lequel l'identification du sous-ensemble est effectuée en fonction de mesures prédéfinies dans l'espace des ondelettes.

8. Procédé selon la revendication 7, dans lequel la détermination des représentations de k spectres de référence plus proches voisins utilise (1340) un sous-ensemble de bandes d'ondelettes disponibles.

9. Procédé selon la revendication 8, dans lequel le sous-ensemble de bandes d'ondelettes disponibles comprend des bandes d'ondelettes de basses et moyennes fréquences, le sous-ensemble ne comprenant que des bandes d'ondelettes comprises entre la bande d'ondelettes la plus basse et la bande d'ondelettes la plus haute.

10. Procédé selon l'une quelconque des revendications précédentes, dans lequel la mesure prédéfinie est une distance de Minkowski.

11. Procédé selon l'une quelconque des revendications précédentes, dans lequel l'empreinte est caractéristique de l'échantillon selon l'un quelconque des aspects suivants : la qualité de l'échantillon, la composition de l'échantillon.

12. Procédé selon l'une quelconque des revendications précédentes, dans lequel le spectre mesuré et les spectres de référence sont obtenus soit par spectroscopie infrarouge, soit par spectroscopie Raman.

13. Procédé selon l'une quelconque des revendications précédentes, dans lequel la bibliothèque de spectres comprend des spectres de référence pour une pluralité de différents états caractéristiques attendus d'un échantillon qui se produisent au cours du traitement de l'échantillon, chaque état caractéristique étant associé à une empreinte respective, et dans lequel, pour chaque sous-processus du traitement, un sous-ensemble approprié de spectres de référence respectifs est identifié au moyen d'une recherche respective des k plus proches voisins.

14. Produit de programme informatique destiné à déterminer la conformité d'une empreinte d'un échantillon avec une empreinte attendue correspondante dudit échantillon, comprenant des instructions lisibles par ordinateur qui, lorsqu'elles sont chargées dans la mémoire d'un dispositif informatique et traitées par un ou plusieurs processeurs du dispositif informatique, amènent le dispositif informatique à exécuter les étapes du procédé selon l'une quelconque des revendications précédentes.

15. Système informatique (100) destiné à déterminer la conformité d'une empreinte d'un échantillon (201) avec une empreinte attendue correspondante dudit échantillon, comprenant des modules fonctionnels adaptés pour exécuter les étapes du procédé selon l'une quelconque des revendications 1 à 13.

FIG. 1

<u>1000</u>

| obtaining measured spectrum obtained by vibrational spectroscopy reflecting sample property | 1100 |

| accessing spectrum library comprising calibrated reference spectra | 1200 |

identifying subset of reference spectra by determining representations of k nearest neighbor reference spectra

> applying wavelet transformation to respective spectra <u>1320</u>

> using subset of available wavelet bands to determine representations of k nearest neighbor reference spectra <u>1340</u>

1300

computing averaged reference spectrum based on identified subset, and computing standard deviation in each data point of the k nearest neighbor reference spectra — 1400

determining difference spectrum by computing difference between measured spectrum and averaged reference spectrum divided by the corresponding standard deviation in each data point — 1500

determining deviating data points where value of difference spectrum exceeds predefined deviation threshold — 1600

determining conformity in accordance with predefined conformity rule based on data points exceeding predefined deviation threshold — 1700

# FIG. 2

FIG. 3

FIG. 4A

FIG. 4B

FIG. 5

FIG. 6

DT1

DT2

DT4

## FIG. 7

$k = 0$     $k = 1$     $k = 2$     $k = 3$

$j = 0$

$j = 1$

$j = 2$

## FIG. 8

EP 4 407 298 B1

FIG. 9A

Spektrum $f$

$j = 0$

$j = 1$

$j = 2$

FIG. 9B

$j = 3$

$j = 4$

$j = 5$

$j = 6$

$j = 7$

$j = 8$

EP 4 407 298 B1

FIG. 10

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **PLUGGE** ; **VAN DER VLIES**. Near-infrared spectroscopy as an alternative to assess compliance of ampicillin trihydrate with compendial specifications. *Journal of Pharmaceutical & Biomedical Analysis*, 1993, 11435-442
- **LÓPEZ et al.** Multivariate screening in food adulteration: Untargeted versus targeted modelling. *Food Chemistry*, 2014, vol. 147, 177-181, https://doi.org/10.1016/j.foodchem.2013.09.139
- **AUDE PESTIEAU**. Towards a real time release approach for manufacturing tablets using NIR spectroscopy. *Journal of Pharmaceutical and Biomedical Analysis*, 2014, vol. 98, ISSN 0731-7085, 60-67

- **CAI**. Treating NIR data with orthogonal discrete wavelet transform: Predicting concentrations of a multi-component system through a small-scale calibration set. *Talanta*, 2008, vol. 77 (2), ISSN 0039-9140, 822-826
- **2019**. *Appendix XVIII : USP 3 S ( FCC 11) Guidance on Developing and Validating Non-Targeted Methods for Adulteration Detection*
- **DAVID B. HIBBERT**. Vocabulary of concepts and terms in chemometrics (IUPAC Recommendations 2016). *Pure Appl. Chem.*, 2016, vol. 88 (4), 407-443
- **U. DEPCZYNSKI** ; **K. JETTER** ; **K. MOLT** ; **A. NIEMOELLER**. Quantitative analysis of near infrared spectra by wavelet coefficient regression using a genetic algorithm. *Chemometrics and Intelligent Laboratory Systems*, 1999, vol. 47, 179-187